# EUROPEAN PATENT APPLICATION

(11) **EP 4 026 559 A1**
(43) Date of publication of application: **13.07.2022**
(21) Application number: 20860453.8
(22) Date of filing: 02.09.2020
(51) Int. Cl.: A61K 39/145, A61P 31/16

(54) **SEASONAL INFLUENZA VACCINE CAPABLE OF INDUCING VIRUS-SPECIFIC ANTIBODY INTO NASAL CAVITY**

(30) Priority: 03.09.2019 JP 2019160280
(71) Applicant: Denka Company Limited, Tokyo 103-8338 (JP)
(72) Inventor: MITSUMATA, Ryotaro, Gosen-shi, Niigata 959-1836 (JP); KANDA, Asumi, Gosen-shi, Niigata 959-1836 (JP); NAKATA, Nagisa, Gosen-shi, Niigata 959-1836 (JP); MIMORI, Shigetaka, Gosen-shi, Niigata 959-1836 (JP)
(74) Representative: Blodig, Wolfgang
(86) International application number: PCT/JP2020/033175
(87) International publication number: WO 2021/045073

(57) **Abstract**

Provided is a seasonal influenza vaccine having a higher efficacy than a split vaccine. A seasonal influenza vaccine which induces virus-specific antibodies in the nasal mucosa, comprises inactivated whole influenza virus particles as an active ingredient, and is to be administered intradermally at dose per administration of 15 µg HA or more per strain as antigen.

## Description

### Field of the Invention

The present invention relates to a highly effective influenza vaccine capable of inducing a virus-specific antibody response in the nasal cavity.

### Background of the Invention

Influenza, which is prevalent in the winter months, is an acute respiratory infection caused by an influenza virus as a pathogen and is spread through droplets from sneezing and the like, or contact with objects to which droplets are attached. During seasons of large epidemic of this influenza, the number of deaths from influenza and its related diseases increases, and this is particularly marked among the elderly. This phenomenon is a common phenomenon in developed countries, and is a serious social problem in Japan, where the proportion of elderly people is rapidly increasing.

One of the most effective ways to prevent this seasonal influenza is a vaccine. The influenza vaccine currently used in Japan is a vaccine using a split antigen vaccine in which virus particles are disrupted with a surfactant or ether. Specifically, a multivalent split vaccine, in which an antigen of each of the A/H1N1 subtype, A/H3N2 subtype, B/Yamagata lineage, and B/Victoria lineage is mixed, is inoculated by subcutaneous administration. This split vaccine is known to show an effectiveness from 40 to 60% when the antigenicity of the vaccine strain matches that of the circulating strain. The efficacy of this split vaccine may seem low at 40%, but there is a U.S. study which reported that if 40% of the population were inoculated, the number of patients with influenza infection would decrease by 21 million, inpatients due to influenza by 130,000, and deaths by 60,000 (Non Patent Literature 1).

On the other hand, there is a demand for an influenza vaccine with higher efficacy in clinical practice, and for this reason, highly effective influenza vaccines were mentioned as vaccines of high priority for development during the Research and Development, Production and Distribution Working Group Meeting of the Subcommittee on Immunization and Vaccines, Health Sciences Council in 2013. In Europe and the United States, highly effective influenza vaccines such as live attenuated intranasal vaccines, adjuvant formulations, and high dose vaccines have already been approved, but these vaccines are intended for elderly people and children who have weak immune response, and are not intended for all age groups. In recent years, there was also a study which reported that the efficacy of live attenuated intranasal vaccines was inferior to that of split vaccines (Non Patent Literature 2), and even for influenza vaccines which were expected to have a high efficacy, its potency has not yet been established.

Therefore, there is currently still a demand for the development of a highly effective influenza vaccine.

The current efficacy standards for seasonal influenza vaccines are defined in the guidelines of the European Medicines Agency (EMA) (Non Patent Literature 3) and are based on serum HI antibody titers or SRH antibody titers. However, influenza viruses are viruses which exhibit pathogenicity by proliferating locally in the respiratory tract, and the mechanism by which the antibodies in the blood show an efficacy against this local infection in the respiratory tract is not clear.

Therefore, the immunological indicators which are directly related to the protection against influenza virus infection or the inhibition of onset must be identified, and highly effective vaccines must be created based on these immunological indicators.

Non Patent Literature 1: Sah P, Medlock J, Fitzpatrick MC, Singer BH, Galvani AP. Optimizing the impact of low-efficacy influenza vaccines. Proc Natl Acad Sci U S A. 2018 May 15;115(20):5151-5156.
Non Patent Literature 2: Jackson ML, Chung JR, Jackson LA, Phillips CH, Benoit J, Monto AS, Martin ET, Belongia EA, McLean HQ, Gaglani M, Murthy K, Zimmerman R, Nowalk MP, Fry AM, Flannery B. Influenza Vaccine Effectiveness in the United States during the 2015-2016 Season. N Engl J Med. 2017 Aug 10;377(6):534-543.
Non Patent Literature 3: Note for guidance on harmonisation of requirements for influenza vaccines. (CPMP/BWP/214/96)

### Summary of the Invention

### Technical Problem

The present invention relates to provision of a seasonal influenza vaccine having a higher efficacy than a split vaccine.

### Solution to Problem

As a result of intensive research, the present inventors found that an inactivated whole particle vaccine against a seasonal influenza virus has a high capacity to induce a virus-specific antibody (IgG) in the nasal mucosa, and that this mucosal IgG effectively acts to eliminate the virus in the upper respiratory tract. Then, as a result of further investigation, the inventors found that intradermal administration of the inactivated whole particle vaccine at a high dose significantly increased the induction of virus-specific antibodies in the nasal mucosa as compared with the administration of conventional split vaccines.

That is, the present invention relates to the following 1) to 8):
1) A seasonal influenza vaccine which induces virus-specific antibodies in the nasal mucosa, comprising inactivated whole influenza virus particles as an active ingredient, wherein 15 µg HA or more per strain per administration is intradermally administered as antigen.
2) The vaccine according to 1), wherein the inactivated whole influenza virus particles comprise either seasonal influenza A virus strain or influenza B virus strain or both thereof.
3) A seasonal influenza vaccine composition for intradermal administration, comprising inactivated whole influenza virus particles in an amount of 15 µg or more per strain in terms of hemagglutinin.
4) The vaccine composition according to 3), not comprising an adjuvant.
5) The vaccine according to 1) or 2), or the vaccine composition according to 3) or 4), which is administered with an intradermal needle or a microneedle.
6) Use of inactivated whole seasonal influenza virus particles for producing an influenza vaccine which induces virus-specific antibodies in the nasal mucosa, wherein the inactivated whole virus particles are administered intradermally at a dose per administration of 15 µg HA or more per strain.
7) An inactivated whole seasonal influenza virus particle for use in influenza vaccine therapy which induces virus-specific antibodies in the nasal mucosa, wherein 15 µg HA or more per strain per administration is intradermally administered.
8) A seasonal influenza vaccine therapy, which induces virus-specific antibodies in the nasal mucosa, wherein inactivated whole influenza virus particles are administered intradermally at a dose per administration of 15 µg HA or more per strain.

### Advantageous Effects of the Invention

The present invention enables the provision of a seasonal influenza vaccine having a high capacity to induce virus-specific antibodies in the nasal mucosa and having an excellent effect of eliminating the virus in the upper respiratory tract, which can greatly contribute to the pharmaceutical industry as the creation of a high value-added prophylactic drug.

### Brief Description of Drawings

[Figure 1] Serum HI antibody titer against B/Phuket/3073/2013 strain on subcutaneous administration of a vaccine.
[Figure 2] IgG titer of nasal lavage fluid against B/Phuket/3073/2013 strain on subcutaneous administration of the vaccine.
[Figure 3] Virus content in nasal lavage fluid 3 days after virus inoculation.
[Figure 4A] IgG titer of nasal lavage fluid against A/Singapore/GP1908/2015 strain.
[Figure 4B] IgG titer of nasal lavage fluid against A/Singapore/INFIMH-16-0019/2016 strain.
[Figure 4C] IgG titer of nasal lavage fluid against B/Phuket/3073/2013 strain.
[Figure 4D] IgG titer of nasal lavage fluid against B/Maryland/15/2016 strain.
[Figure 5] Neutralizing antibody titer of nasal lavage fluid against A/Singapore/GP1908/2015 strain.

### Detailed Description of the Invention

The following describes in detail the preferred embodiments of the present invention. However, the present invention is not limited to the following embodiments.

In the present invention, "seasonal influenza vaccine" means a vaccine for seasonal influenza, which contains at least an antigen of either the seasonal influenza A virus or influenza B virus. That is, the seasonal influenza vaccine of the present invention may be a monovalent vaccine containing only one of the seasonal influenza A virus or influenza B virus, or a multivalent vaccine containing both of them, but preferably, it is a trivalent or higher valent vaccine containing at least three or more influenza virus strains, for example, two influenza A virus strains and one or two influenza B virus strains, and preferably includes a quadrivalent vaccine. More preferably, it includes a quadrivalent vaccine containing two A strains (A/H1N1 and A/H3N2 subtypes) and two B strains (B/Victoria and B/Yamagata lineages).

In the present invention, when using the term "influenza virus", it refers to the seasonal influenza A virus or influenza B virus, or both. Influenza virus also includes all currently known subtypes, as well as subtypes to be isolated and identified in the future.

The influenza virus strain used in the preparation of the vaccine of the present invention may be a strain isolated from an infected animal or patient, or a recombinant virus established in cultured cells by genetic engineering.

"Inactivated whole influenza virus particle", the antigen of the seasonal influenza vaccine of the present invention, refers to a virus particle which is obtained by culturing a seasonal influenza virus while retaining the morphology of the virus, and has been subjected to an inactivation treatment.

The inactivated whole influenza virus particle of the present invention can be prepared using the embryonated chicken egg method or cell culture method.

The "embryonated chicken egg method" refers to a method in which a virus strain is inoculated into incubated embryonated chicken eggs and cultured, and then the virus suspension is clarified, concentrated, purified, and inactivated to obtain a virus solution containing virus particles. The "cell culture method" refers to a method in which a virus strain is inoculated into cultured cells and cultured, and then a virus solution containing virus particles is obtained from the culture supernatant in the same way as the above embryonated chicken egg method.

Here, the culture is performed at 30 to 37°C for about 1 to 7 days, preferably at 33 to 35°C for about 2 days after inoculation of the influenza virus strain. After the culture is completed, the virus suspension (infected allantoic fluid or infected cell culture supernatant) is collected, and centrifugation or filtration is performed for clarification. Then, a barium salt adsorption-elution reaction or ultrafiltration is performed for concentration. The virus purification can be performed by using ultracentrifugation such as sucrose density gradient centrifugation or other means such as liquid chromatography.

The purified virus solution is subjected to an inactivation treatment. Examples of the virus inactivation method include formalin treatment, ultraviolet irradiation, and treatments with beta-propiolactone, binary ethylenimine, and the like.

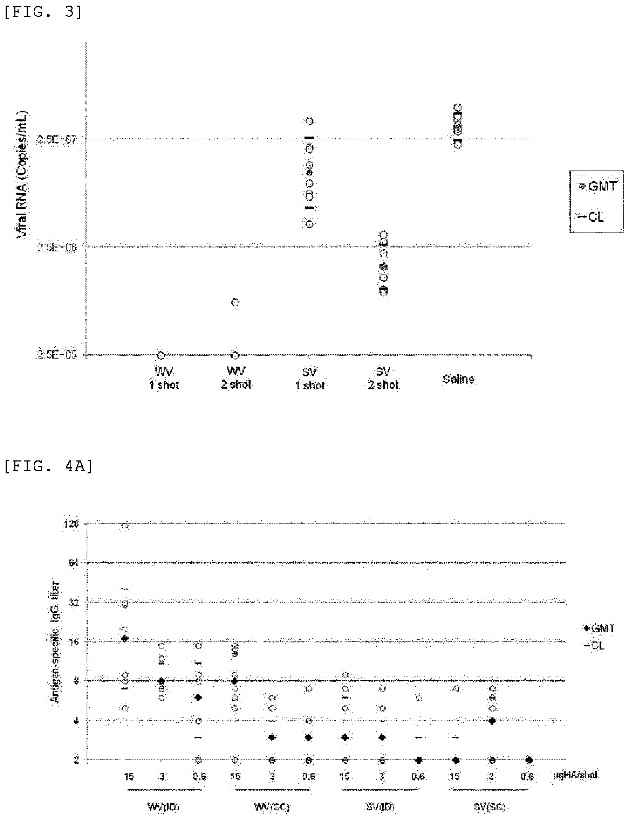

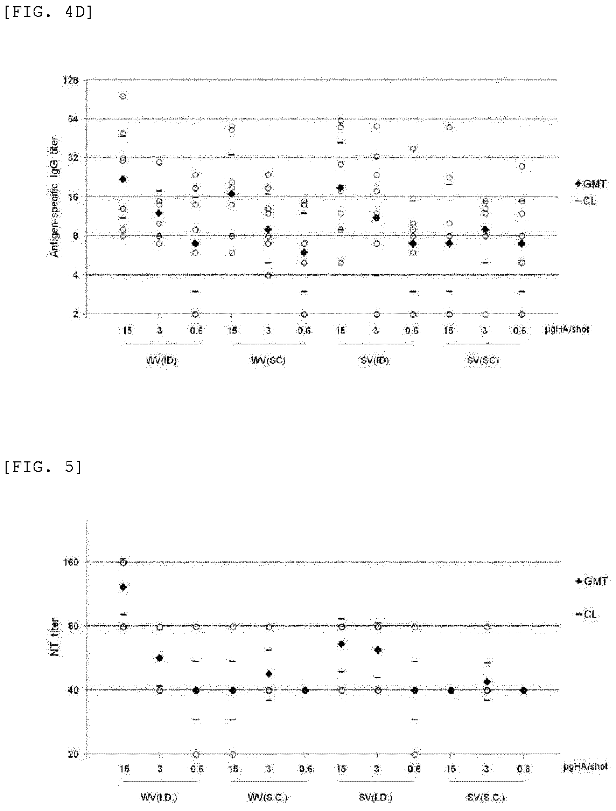

As shown in the examples described below, when inactivated whole particles from four influenza viruses are administered intradermally in an amount of 15 µg or more as hemagglutinin (HA) per strain, the capacity to induce virus-specific antibodies in the nasal cavity is significantly enhanced, and a higher induction of antibodies can be achieved earlier as compared with intradermal and subcutaneous administrations of a split vaccine (Figure 4A to 4D). Such a vaccine with good efficiency in inducing virus-specific antibodies in the nasal cavity has a high virus neutralizing activity and a high capacity to eliminate the virus in the nasal cavity (Figure 5).

Therefore, intradermal administration of 15 µg HA or more per strain of inactivated whole influenza virus particles can be a vaccine therapy for seasonal influenza which induces virus-specific antibodies in the nasal mucosa. In other words, inactivated whole particles of influenza virus can be a seasonal influenza vaccine which induces virus-specific antibodies in the nasal mucosa and is administered intradermally at a dose of 15 µg HA or more per strain.

Here, "inducing virus-specific antibodies in the nasal mucosa means" means, for example, that the neutralizing antibody titer produced against the vaccinated influenza virus antigens in the nasal mucosa is 80 or more, preferably 160 or more. Here, the neutralizing antibody titer can be measured by assessing the inhibitory activity on the cytotoxicity of the virus against MDCK cells.

The seasonal influenza vaccine of the present invention can be formulated as a vaccine composition for intradermal administration (hereinafter referred to as the "vaccine composition of the present invention").

In the present invention, "intradermal administration" refers to as administration into the dermis of the skin and does not mean that the administered vaccine composition is localized only in the dermis. Since the thickness of the dermal layer varies among individuals and even at different sites within an individual, the intradermally administered vaccine composition may be present only or primarily in the skin, or it may be present in the epidermis, which are included in the intradermal administration of the present invention.

The skin is composed of three layers: the epidermis, the dermis, and the subcutaneous tissue. The epidermis is the layer from 0.05 to 0.2 mm from the surface of the skin, the dermis is a layer from 1.5 to 3.0 mm located between the epidermis and the subcutaneous tissue, and the subcutaneous tissue is a layer from 3.0 to 10.0 mm located on the inner side of the dermis. For intradermal administration which is an administration into the dermis, a thinner and shorter needle than the needles for subcutaneous administration is used, and thus the pain and psychological burden at the needle pricking can be reduced compared with the existing vaccines administered subcutaneously or intramuscularly.

In addition to the inactivated whole influenza virus particles, the vaccine composition of the present invention may further contain a pharmaceutically acceptable carrier. Examples of the carrier include carriers commonly used in the production of vaccines, and specific examples thereof include a buffer, an emulsifier, a preservative (for example, thimerosal), an isotonic agent, a pH adjuster, an inactivating agent (for example, formalin), an adjuvant and an immunostimulant. An adjuvant is a substance which, when administered with an antigen, enhances the immune response to the antigen. However, the vaccine composition of the present invention does not necessarily require the addition of an adjuvant since the inactivated whole influenza virus particles themselves, which are the vaccine antigen, have a high capacity to induce antibodies as described above, and can be made into a composition without adjuvant.

The content of inactivated whole influenza virus particles in the vaccine composition of the present invention is 15 µg or more per virus strain in terms of hemagglutinin, that is, 15 µg HA or more per strain, preferably from 15 to 60 µg HA/strain, and more preferably from 15 to 21 µg HA/strain. Note that the hemagglutinin content is the value obtained by measuring with a test method defined by the WHO or national standards, such as the single radial immunodiffusion test method.

A dose per administration of the vaccine composition of the present invention should be, as antigen (inactivated whole influenza virus particles), 15 µg HA or more per strain of virus, and can be increased as appropriate in consideration of the age, sex, weight, and the like of the subject, but preferably from 15 to 60 µg HA/strain, more preferably from 15 to 21 µg HA/strain, and even more preferably 15 µg HA/strain, is administered once or more. Preferably, it is administered in multiple doses, in which case it is preferably administered at an interval from 1 to 4 weeks.

The volume per intradermal administration of the vaccine composition of the present invention is determined according to the site of administration and the administration device, but is usually from about 0.05 to 0.5 mL, preferably from 0.1 to 0.3 mL, and more preferably 0.2 mL

As an administration device for intradermal administration of the vaccine composition of the present invention, an intradermal needle or a microneedle capable of intradermal administration can be used, but for example, the protruding length of the needle tube of the intradermal needle or the microneedle is from 0.9 mm to 1.5 mm, preferably from 1.0 to 1.2 mm, and more preferably 1.2 mm.

The intradermal administration device may have one needle tube, or a plurality of needle tubes, but is preferably an intradermal needle having from one to three needle tubes, and more preferably three needle tubes.

The subject who receives the vaccine composition of the present invention includes humans and mammals other than humans, but humans are preferable. Examples of the mammals other than humans include rats, guinea pigs, rabbits, pigs, cows, horses, goats, sheep, dogs, cats, rhesus monkeys, cynomolgus monkeys, orangutans, and chimpanzees.

### Examples

Hereinafter, the present invention will be described more specifically with examples, but the present invention is not in any way limited thereto.

### Reference Example 1: Induction of virus-specific IgG in the nasal cavity by inactivated whole particle vaccine

A stock solution of the B/Yamagata lineage (B/Phuket/3073/2013 strain) from the influenza HA vaccine "SEIKEN" was used as a split antigen, and adjusted with 6.7 mM phosphate buffered saline containing 1 w/w% sucrose (pH 7.2) to have a hemagglutinin amount of 15 µg HA per 0.2 mL, and the adjusted solution was used as a split vaccine. In addition, inactivated whole particle antigen of the B/Yamagata lineage (B/Phuket/3073/2013 strain) prepared as described below was also adjusted in the same manner with 6.7 mM phosphate buffered saline containing 1 w/w% sucrose (pH 7.2) to have a hemagglutinin amount of 15 µg HA per 0.2 mL, and the adjusted solution was used as an inactivated whole particle vaccine.

The preparation of the inactivated whole particle antigen used in the present reference example is as described below. A virus of the B/Phuket/3073/2013 strain was inoculated into the chorioallantoic cavity of 12-day-old embryonated chicken eggs, and the chorioallantoic fluid was collected after 3 days of incubation. The collected chorioallantoic fluid was clarified by filter filtration, then adsorbed on barium sulfate salt, and eluted with a 12% sodium citrate solution to recover the influenza virus. The recovered virus was further purified by replacing the solution with 6.7 mM phosphate buffered saline (pH 7.2) by ultrafiltration, and after the buffer replacement, recovering the fraction containing the influenza virus by sucrose density gradient centrifugation. To this purified influenza virus, beta-propiolactone as an inactivating agent was added to obtain a final concentration of 0.05%, followed by allowing the mixture to react at 4°C for 24 hours to inactivate the infectivity of the influenza virus. After this inactivation reaction, the buffer was replaced with 6.7 mM phosphate buffered saline containing 1 w/w% sucrose (pH 7.2) by ultrafiltration (MWCO: 100,000), and the resultant was used as an inactivated whole particle vaccine.

The administration solution prepared as described above (Table 1) was administered subcutaneously to BALB/c mice (female, 5 weeks old) at a dose of 0.2 mL per mouse, and whole blood was collected 21, 42, and 63 days after administration (collected from 8 mice per time point). In the same manner, each administration solution was administered twice at a 21-day interval, and whole blood was collected 42 and 62 days after the first dose (21 and 42 days after the second dose, respectively).

After collecting the whole blood, a pipetman was inserted from the pharyngeal side of the maxilla, to pour D-PBS containing 0.175% BSA and protease inhibitors (Thermo Fisher Scientific), and the solution recovered from the external nostrils was used as the nasal lavage fluid. The collected blood was centrifuged to prepare serum, and the serum HI antibody titer against the B/Phuket/3073/2013 strain was measured. For the nasal lavage fluid, the titer of IgG binding to the B/Phuket/3073/2013 strain was measured.

**[Table 1]**

| Antigen Morphology | Vaccine Strain | Frequency of Administration | Sampling Day* (Days) | Route of Administration | N |
|---|---|---|---|---|---|
| Inactivated Whole Particle Vaccine (W.V.) | B/Phuket/3073/2013 | 1 | 21 | Subcutaneous | 8 |
| | | | 42 | Subcutaneous | 8 |
| | | | 63 | Subcutaneous | 8 |
| | | 2 | 42 | Subcutaneous | 8 |
| | | | 63 | Subcutaneous | 8 |
| Split Vaccine (S.V.) | B/Phuket/3073/2013 | 1 | 21 | Subcutaneous | 8 |
| | | | 42 | Subcutaneous | 8 |
| | | | 63 | Subcutaneous | 8 |
| | | 2 | 42 | Subcutaneous | 8 |
| | | | 63 | Subcutaneous | 8 |

| | | | | | |
|---|---|---|---|---|---|
| *Counted from the date of the first dose | | | | | |

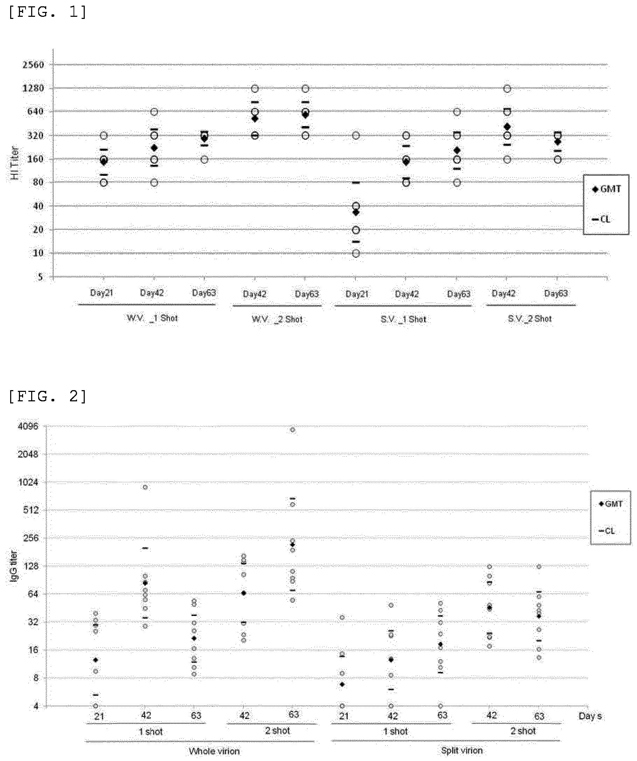

The serum HI antibody titers against the B/Phuket/3073/2013 strain are as shown in Figure 1. In comparison among the single-dose groups, the antibody titers in the split vaccine administration group were lower on Day 21 due to a slower induction of antibodies in the split vaccine compared with the inactivated whole particle vaccine, but the antibody titers in the split vaccine administration group gradually increased on Days 42 and 63, and after Day 42, the antibody titers in both vaccine administration groups were similar. In the double-dose groups, the inactivated whole particle vaccine had similar antibody titers on Days 42 and 63, but a tendency to slightly decrease was found for the split vaccine from Days 42 to 63. Therefore, on Day 42, the HI antibody titers for the inactivated whole particle vaccine and the split vaccine were equivalent, but on Day 63, they tended to be higher for the inactivated whole particle vaccine.

The virus-specific IgG titers of the nasal lavage fluids are shown in Figure 2. In comparison among the single-dose groups, the IgG titers gradually increased from Day 21 to 63 in the split vaccine administration group, whereas the antibody titers were relatively low. By contrast, the inactivated whole particle vaccine administration group produced a bell shape with a peak at Day 42, showing a different trend from the HI antibody titers. Therefore, while the serum HI antibody titers on Day 42 and Day 63 were similar for both vaccines, the virus-specific IgG titers of the nasal lavage fluids showed a significantly higher induction of antibodies in the inactivated whole particle vaccine administration group than in the split vaccine administration group on Day 42.

In the double-dose groups, while the virus-specific IgG titers in the nasal lavage fluids on Day 42 also increased in the split vaccine administration group, the antibody titers were lower compared with the inactivated whole particle vaccine administration group. Furthermore, when compared on Day 63 (42 days after the second dose), the antibody titers tended to increase in the inactivated whole particle vaccine administration group, while those in the split vaccine group tended to decrease, which suggests that the inactivated whole particle vaccine was superior in terms of the durability of the virus-specific IgG in the nasal mucosa.

### Reference Example 2: Virus Challenge Test

In the same manner as in Reference Example 1, the split antigen or inactivated whole particle antigen of the B/Victoria lineage (B/Texas/2/2013 strain) was adjusted with 6.7 mM phosphate buffered saline containing 1 w/w% sucrose (pH 7.2) to have a hemagglutinin amount of 15 µg HA per 0.2 mL, and the resultant was used as a split vaccine or inactivated whole particle vaccine.

The administration solutions prepared as described above (Table 2) were administered subcutaneously to BALB/c mice (female, 5 weeks old) at a dose of 0.2 mL per mouse once or twice at a 3-week interval. To the control group, saline (Otsuka Pharmaceutical Factory) was administered subcutaneously twice at a 3-week interval. Forty-two days after the first dose, the B/Brisbane/60/2008 strain of the B/Victoria lineage was inoculated into the nasal cavity under isoflurane anesthesia at 1 × 10⁴ TCID₅₀/10 µL/head. Three days after virus inoculation, each mouse was euthanized by exsanguination, and then the nasal lavage fluids were collected in the same manner as in Reference Example 1.

The amount of virus contained in the collected nasal lavage fluids was measured by qPCR to compare and evaluate the capacity to eliminate the virus in the nasal mucosa.

**[Table 2]**

| Antigen Morphology | Vaccine Strain | Frequency of Administration | Dose Administered (µgHA/head) | Volume Administered | Sampling Date | N |
|---|---|---|---|---|---|---|
| Inactivated Whole Particle Vaccine (W.V.) | B/Texas/2/2013 | 1 | 15 | 0.2 mL | Day 45 | 8 |
| Inactivated Whole Particle Vaccine (W.V.) | B/Texas/2/2013 | 2 | 15 | 0.2 mL | Day 45 | 8 |
| Split Vaccine (S.V.) | B/Texas/2/2013 | 1 | 15 | 0.2 mL | Day 45 | 8 |
| Split Vaccine (S.V.) | B/Texas/2/2013 | 2 | 15 | 0.2 mL | Day 45 | 8 |
| Saline | - | 2 | - | 0.2 mL | Day 45 | 8 |

The virus content of the nasal lavage fluids three days after virus inoculation is as shown in Figure 3. By subcutaneous administration of the inactivated whole particle vaccine, the virus-specific IgG in the nasal mucosa increased 42 days after the single dose and after two doses (Reference Example 1), and in the virus challenge test, almost no virus was detected when inoculated at the above time points. On the other hand, with the subcutaneous administration of the split vaccine, it was confirmed that 42 days after the single dose, the virus content was slightly reduced compared with the control group, but the virus content of the nasal lavage fluid was high, probably due to low virus-specific IgG titers in the nasal mucosa. However, with the subcutaneous administration of the split vaccine, the virus-specific IgG titers in the nasal mucosa on Day 42 also increased by administering two doses (Reference Example 1), and therefore the virus contents in the double-dose groups were less than one-tenth compared with the control, which confirms a significant decrease in virus content in the nasal mucosa.

These results suggest that the induction of high levels of virus-specific IgG titers in the nasal mucosa is associated with the efficacy of the influenza vaccine.

### Example 1: Evaluation of antibody induction in the nasal mucosa by intradermal administration of influenza vaccine

Inactivated whole particle vaccines and split vaccines were prepared using split antigens and inactivated whole particle antigens of four strains (A/H1N1, A/H3N2, B/Yamagata lineage, and B/Victoria lineage) prepared in the same manner as in Reference Example 1. The vaccines for intradermal administration were adjusted to have a hemagglutinin amount of 15, 3, or 0.6 µg HA per 0.2 mL, and the vaccines for subcutaneous administration were adjusted to have a hemagglutinin amount of 15, 3, or 0.6 µg HA per 0.5 mL, with 6.7 mM phosphate buffered saline containing 1 w/w% sucrose (pH 7.2).

The administration solutions prepared as described above (Table 3-1 and Table 3-2) were administered to the dorsal part of SD rats (male, 9 weeks old) twice at a 3-week interval, at 0.2 mL per mouse for intradermal administration and at 0.5 mL per mouse for subcutaneous administration. Twenty-one days after the second dose, blood removal was performed under isoflurane anesthesia, and then the nasal lavage fluids were collected. As in Reference Example 1, the method for collecting the nasal lavage fluid was to insert a pipetman from the pharyngeal side of the maxilla, to pour D-PBS containing 0.175% BSA and protease inhibitors (Thermo Fisher Scientific), and the solution recovered from the external nostrils was used as the nasal lavage fluid. Note that for intradermal administration, a Paskin triple-tip needle (Nanbu Plastics, 34G, 1.2mm) was used.

The recovered nasal lavage fluids were used to measure the titers of virus-specific IgG bound to each vaccine strain and to measure the neutralizing antibody titers against the A/Singapore/GP1908/2015 strain.

**[Table 3-1]**

| Antigen Morphology | Vaccine Strain | Dose Administered (µgHA/strain/head) | Volume Administered (mL) | Route of Administration | N |
|---|---|---|---|---|---|
| Inactivated Whole Particle Vaccine (W.V.) | A/Singapore/GP1908/2015 | 15 | 0.2 | Intradermal | 8 |
| | A/Singapore/INFIMH-16-0019/2016 | | | | |
| | B/Phuket/3073/2013 | | | | |
| | B/Maryland/15/2016 | | | | |
| Inactivated Whole Particle Vaccine (W.V.) | A/Singapore/GP1908/2015 | 3 | 0.2 | Intradermal | 8 |
| | A/Singapore/INFIMH-16-0019/2016 | | | | |
| | B/Phuket/3073/2013 | | | | |
| | B/Maryland/15/2016 | | | | |
| Inactivated Whole Particle Vaccine (W.V.) | A/Singapore/GP1908/2015 | 0.6 | 0.2 | Intradermal | 8 |
| | A/Singapore/INFIMH-16-0019/2016 | | | | |
| | B/Phuket/3073/2013 | | | | |
| | B/Maryland/15/2016 | | | | |
| Inactivated Whole Particle Vaccine (W.V.) | A/Singapore/GP1908/2015 | 15 | 0.5 | Subcutaneous | 8 |
| | A/Singapore/INFIMH-16-0019/2016 | | | | |
| | B/Phuket/3073/2013 | | | | |
| | B/Maryland/15/2016 | | | | |
| Inactivated Whole Particle Vaccine (W.V.) | A/Singapore/GP1908/2015 | 3 | 0.5 | Subcutaneous | 8 |
| | A/Singapore/INFIMH-16-0019/2016 | | | | |
| | B/Phuket/3073/2013 | | | | |
| | B/Maryland/15/2016 | | | | |
| Inactivated Whole Particle Vaccine (W.V.) | A/Singapore/GP1908/2015 | 0.6 | 0.5 | Subcutaneous | 8 |
| | A/Singapore/INFIMH-16-0019/2016 | | | | |
| | B/Phuket/3073/2013 | | | | |
| | B/Maryland/15/2016 | | | | |

**[Table 3-2]**

| Antigen Morphology | Vaccine Strain | Dose Administered (µgHA/strain/head) | Volume Administered (mL) | Route of Administration | N |
|---|---|---|---|---|---|
| Split Vaccine (S.V.) | A/Singapore/GP1908/2015 | 15 | 0.2 | Intradermal | 8 |
| | A/Singapore/INFIMH-16-0019/2016 | | | | |
| | B/Phuket/3073/2013 | | | | |
| | B/Maryland/15/2016 | | | | |
| Split Vaccine (S.V.) | A/Singapore/GP1908/2015 | 3 | 0.2 | Intradermal | 8 |
| | A/Singapore/INFIMH-16-0019/2016 | | | | |
| | B/Phuket/3073/2013 | | | | |
| | B/Maryland/15/2016 | | | | |
| Split Vaccine (S.V.) | A/Singapore/GP1908/2015 | 0.6 | 0.2 | Intradermal | 8 |
| | A/Singapore/INFIMH-16-0019/2016 | | | | |
| | B/Phuket/3073/2013 | | | | |
| | B/Maryland/15/2016 | | | | |
| Split Vaccine (S.V.) | A/Singapore/GP1908/2015 | 15 | 0.5 | Subcutaneous | 8 |
| | A/Singapore/INFIMH-16-0019/2016 | | | | |
| | B/Phuket/3073/2013 | | | | |
| | B/Maryland/15/2016 | | | | |
| Split Vaccine (S.V.) | A/Singapore/GP1908/2015 | 3 | 0.5 | Subcutaneous | 8 |
| | A/Singapore/INFIMH-16-0019/2016 | | | | |
| | B/Phuket/3073/2013 | | | | |
| | B/Maryland/15/2016 | | | | |
| Split Vaccine (S.V.) | A/Singapore/GP1908/2015 | 0.6 | 0.5 | Subcutaneous | 8 |
| | A/Singapore/INFIMH-16-0019/2016 | | | | |
| | B/Phuket/3073/2013 | | | | |
| | B/Maryland/15/2016 | | | | |

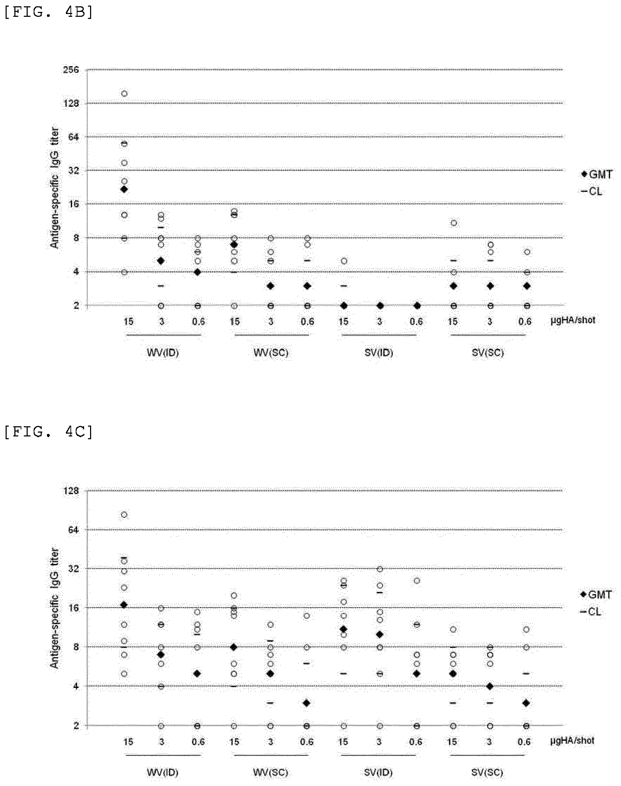

The results for the virus-specific IgG titers of the nasal lavage fluids are as shown in Figures 4A to 4D. The IgG titers against the A/Singapore/GP1908/2015 strain (A/H1N1 subtype) and the A/Singapore/INFIMH-16-0019/2016 strain (A/H3N2 subtype) confirmed the induction at high doses in the group of intradermal administration of inactivated whole particle vaccine or the group of subcutaneous administration of inactivated whole particle vaccine, with almost no induction in the other groups (Figures 4A and 4B). Among the groups in which the induction of virus-specific IgG was confirmed in the nasal mucosa, the highest induction efficiency was observed in the group which intradermally received 15 µg HA/strain/head of the inactivated whole particle vaccine. Therefore, the administration of an inactivated whole particle vaccine is effective for inducing virus-specific IgG against type A viruses in the nasal mucosa, and in particular, high antibody induction can be achieved by administering intradermally an inactivated whole particle vaccine at a high dose.

For the IgG titers against the B/Phuket/3073/2013 strain and the B/Maryland/15/2016 strain, the highest induction was shown in the group of intradermal administration of the inactivated whole particle vaccine (Figures 4C and 4D). For type B, induction of virus-specific IgG was observed also in the nasal mucosa in the group of intradermal administration of the split vaccine, but the induction efficiency was found to be the lowest in the group of subcutaneous administration (SV (S.C.)) of the split vaccine, which is identical to the current influenza vaccine. Therefore, intradermal administration of an inactivated whole particle vaccine was considered to be the one efficiently capable of inducing specific IgG against viruses of both type A and B in the nasal mucosa.

Figure 5 shows the results of the neutralizing antibody titers against the A/Singapore/GP1908/2015 strain (A/H1N1 subtype) in the nasal lavage fluids. However, high virus neutralizing activity was shown only in the group which intradermally received 15 µg HA/strain/head of inactivated whole particle vaccine.

Therefore, also in the results for the virus-specific IgG titers in the nasal mucosa, the intradermal administration of the inactivated whole particle vaccine (15 µg HA/strain/head) had a high IgG titer against any strain and also had the highest virus neutralizing activity. That is, intradermal administration of a high dose of an inactivated whole particle vaccine increases the induction of antibodies which eliminate the virus in the nasal mucosa, which makes it a more effective influenza vaccine than the current vaccines.

## Claims

1. A seasonal influenza vaccine which induces virus-specific antibodies in the nasal mucosa, comprising inactivated whole influenza virus particles as an active ingredient, wherein 15 µg HA or more per strain per administration is intradermally administered as antigen.

2. The vaccine according to claim 1, wherein the inactivated whole influenza virus particles comprise either seasonal influenza A virus strain and influenza B virus strain or both thereof.

3. A seasonal influenza vaccine composition for intradermal administration, comprising inactivated whole influenza virus particles in an amount of 15 µg or more per strain in terms of hemagglutinin.

4. The vaccine composition according to claim 3, not comprising an adjuvant.

5. The vaccine according to claim 1 or 2, or the vaccine composition according to claim 3 or 4, which is administered with an intradermal needle or a micro needle.

6. Use of inactivated whole seasonal influenza virus particles for producing an influenza vaccine which induces virus-specific antibodies in the nasal mucosa, wherein the inactivated whole virus particles are administered intradermally at a dose per administration of 15 µg HA or more per strain.

7. An inactivated whole seasonal influenza virus particle for use in influenza vaccine therapy which induces virus-specific antibodies in the nasal mucosa, wherein 15 µg HA or more per strain per administration is intradermally administered.

8. A seasonal influenza vaccine therapy which induces virus-specific antibodies in the nasal mucosa, wherein inactivated whole influenza virus particles are administered intradermally at a dose per administration of 15 µg HA or more per strain.
